(19) Europäisches Patentamt
European Patent Office
Office européen des brevets

(11) Veröffentlichungsnummer: **0 315 019**
**A2**

# (12) EUROPÄISCHE PATENTANMELDUNG

(21) Anmeldenummer: **88117703.4**

(22) Anmeldetag: **25.10.88**

(51) Int. Cl.[4]: **C07D 211/90 , A61K 31/44**

(30) Priorität: **04.11.87 DE 3737341**

(43) Veröffentlichungstag der Anmeldung:
**10.05.89 Patentblatt 89/19**

(84) Benannte Vertragsstaaten:
**AT BE CH DE ES FR GB GR IT LI LU NL SE**

(71) Anmelder: **BAYER AG**
**Konzernverwaltung RP Patentabteilung**
**D-5090 Leverkusen 1 Bayerwerk(DE)**

(72) Erfinder: **Franckowiak, Gerhard, Dr.**
**Henselweg 10**
**D-5600 Wuppertal 1(DE)**
Erfinder: **Stoltefuss, Jürgen, Dl.**
**Parkstrasse 20**
**D-5657 Haan 2(DE)**
Erfinder: **Bechem, Martin, Dr.**
**Obere Bergerheide 4**
**D-5600 Wuppertal 1(DE)**
Erfinder: **Gross, Rainer, Prof. Dr.**
**Platzhofstrasse 23**
**D-5600 Wuppertal 1(DE)**
Erfinder: **Kayser, Michael, Dr.**
**Walter-Flex-Strasse 18**
**D-5090 Leverkusen 1(DE)**
Erfinder: **Hebisch, Siegbert, Dr.**
**Herzogstrasse 129**
**D-4200 Oberhausen 11(DE)**
Erfinder: **Schramm, Matthias, Dr.**
**Paffrather Strasse 38**
**D-5000 Köln 80(DE)**

## (54) Neue Fluormethoxyphenyl-dihydropyridine, Verfahren zur Herstellung und ihre Verwendung in Arzneimitteln.

(57) Die vorliegende Erfindung betrifft neue Fluormethoxyphenyl-dihydropyridine der allgemeinen Formel I

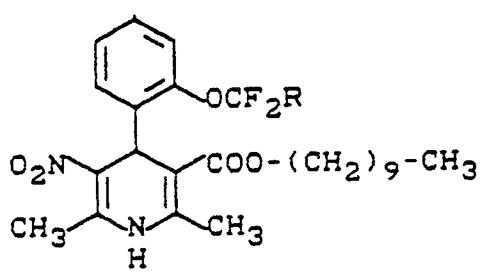

(I)

in welcher R für Wasserstoff oder Fluor steht, mehrere Verfahren zu ihrer Herstellung sowie ihre Verwendung in Arzneimitteln, insbesondere in kreislaufbeeinflussenden Mitteln mit positiv inotroper Wirkung.

EP 0 315 019 A2

## Neue Fluormethoxyphenyl-dihydropyridine, Verfahren zur Herstellung und ihre Verwendung in Arzneimitteln

Die vorliegende Erfindung betrifft neue Fluormethoxyphenyl-dihydropyridine, mehrere Verfahren zu ihrer Herstellung sowie ihre Verwendung in Arzneimitteln, insbesondere in kreislaufbeeinflussenden Mitteln mit positiv inotroper Wirkung.

Est ist bereits bekannt, daß 1,4-Dihydropyridine gefäßerweiternde Eigenschaften besitzen und als Coronarmittel und Antihypertensiva verwendet können (vgl. Brit. Patent 1 173 062 und 1 358 951; DE-OS 2 629 892 und 27 52 820). Weiterhin ist bekannt, daß 1,4-Dihydropyridine eine Hemmung der Kontraktionskraft von glatten und cardialen Muskeln bewirken und zur Behandlung von Coronar- und Gefäßerkrankungen eingesetzt werden können (vgl. Fleckenstein, Ann. Rev. Pharmcol, Toxicol. 17, 149 - 166 (1977)).

Es ist außerdem bekannt, daß 3-Nitro-dihydropyridine im allgemeinen neben der positiv inotropen Herzwirkung den Nachteil einer unerwünschten konstringierenden Wirkung an den Koronargefäßen zeigen können [Schramm et al., Nature 303, 535-537 (1983)] (siehe auch DE-OS 3 447 169).

Bei Kenntnis dieser Eigenschaften der Dihydropyridine war es nicht vorhersehbar, daß die erfindungsgemäßen Verbindungen eine kontraktionskraftverstärkende, am Herzmuskel positiv inotrope Wirkung mit weitgehend gefäßneutralem Verhalten besitzen.

Die Erfindung betrifft neue Fluormethoxyphenyl-dihydropyridine der allgemeinen Formel (I),

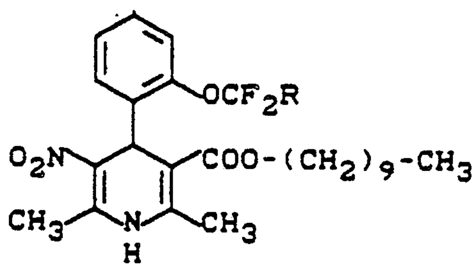

(I)

in welcher
R - für Wasserstoff oder Fluor steht.

Die erfindungsgemäßen Verbindungen der Formel (I), in welcher
R die oben angegebene Bedeutung hat,
erhält man, indem man

[A] den Aldehyd der Formel (II)

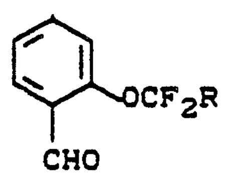

(II)

und Nitroaceton der Formel (III)

$H_3C-CO-CH_2-NO_2$ (III)

oder deren Knoevenagel-Kondensationsprodukt der Formel (IV),

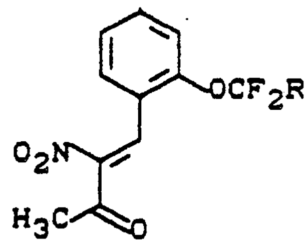

(IV)

in welcher R die oben angegebene Bedeutung hat, und den Aminocrotonsäureester der Formel (V)

$CH_3-\underset{\substack{|\\ NH_2}}{C}= CH-COO-(CH_2)_9-CH_3$ (V)

gegebenenfalls in Anwesenheit inerter Lösemittel umsetzt, oder indem man

[B] das Dihydropyridin der Formel (VI)

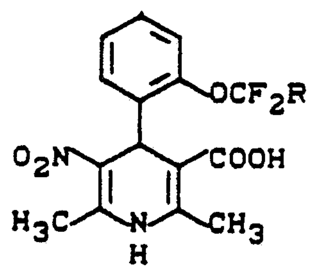

(VI)

in welcher R die oben angegebene Bedeutung hat, mit einem gegebenenfalls reaktiven Decanol der Formel (VII)

$H_3C-(CH_2)_9-Y$ (VII)

in welcher Y für Hydroxyl oder eine aktivierende Gruppe wie Halogen, $-O-SO_2O-(CH_2)_9-CH_3$ oder ähnliche steht, nach bekannten Methoden, gegenbenfalls über ein reaktives Säurederivat, umsetzt, oder indem man

[C] den Aldehyd der Formel (II) und den Acetessigester der Formel (VIII)

$H_3C-CO-CH_2-COO-(CH_2)_9-CH_3$ (VIII)

bzw. deren Knoevenagel-Kondensationsprodukte der Formel (IX)

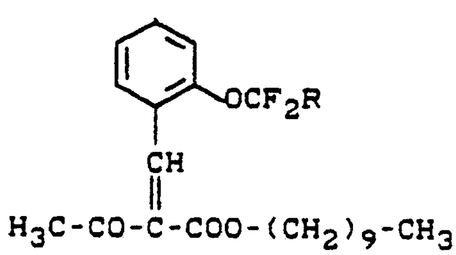

(IX)

in welchen R die oben angegebene Bedeutung hat, mit Nitroaceton und Ammoniumsalzen wie z.B. Ammoniumacetat, gegebenenfalls in Anwesenheit inerter

Lösemittel, umsetzt und die erhaltenen Dihydropyridine gegebenfalls nach üblichen Methoden, z.B. durch Chromatographie, über chirale stationäre Phasen auftrennt.

Je nach Art der verwendeten Ausgangsstoffe läßt sich die Synthese der racemischen Verbindungen nach Verfahren A bis C durch folgende Formelschemata verdeutlichen:

[A]

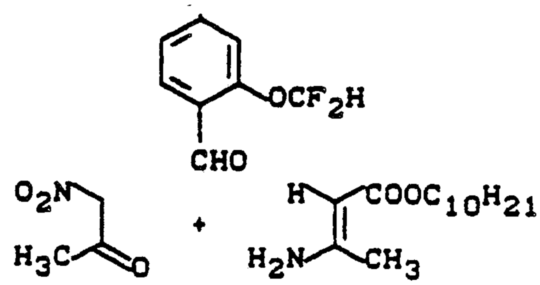

bzw.

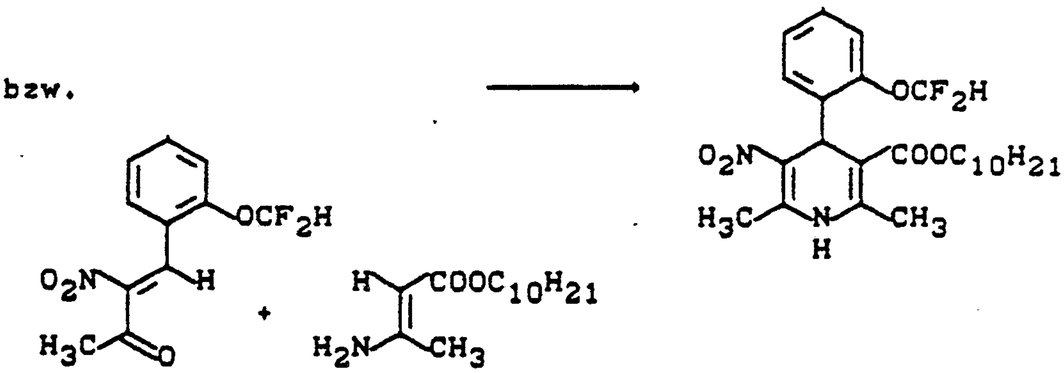

[B]

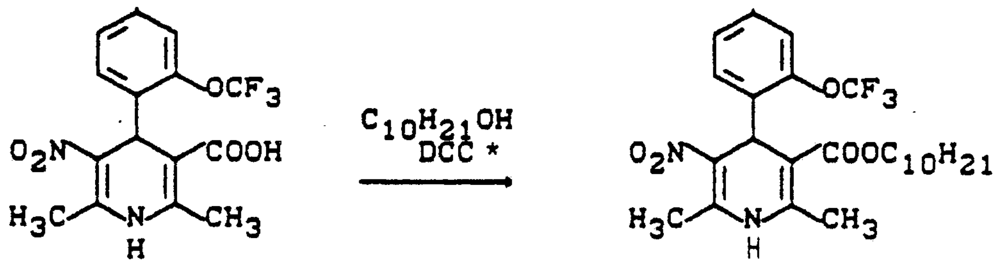


* = Dicyclohexyl-carbodiimid

[C]

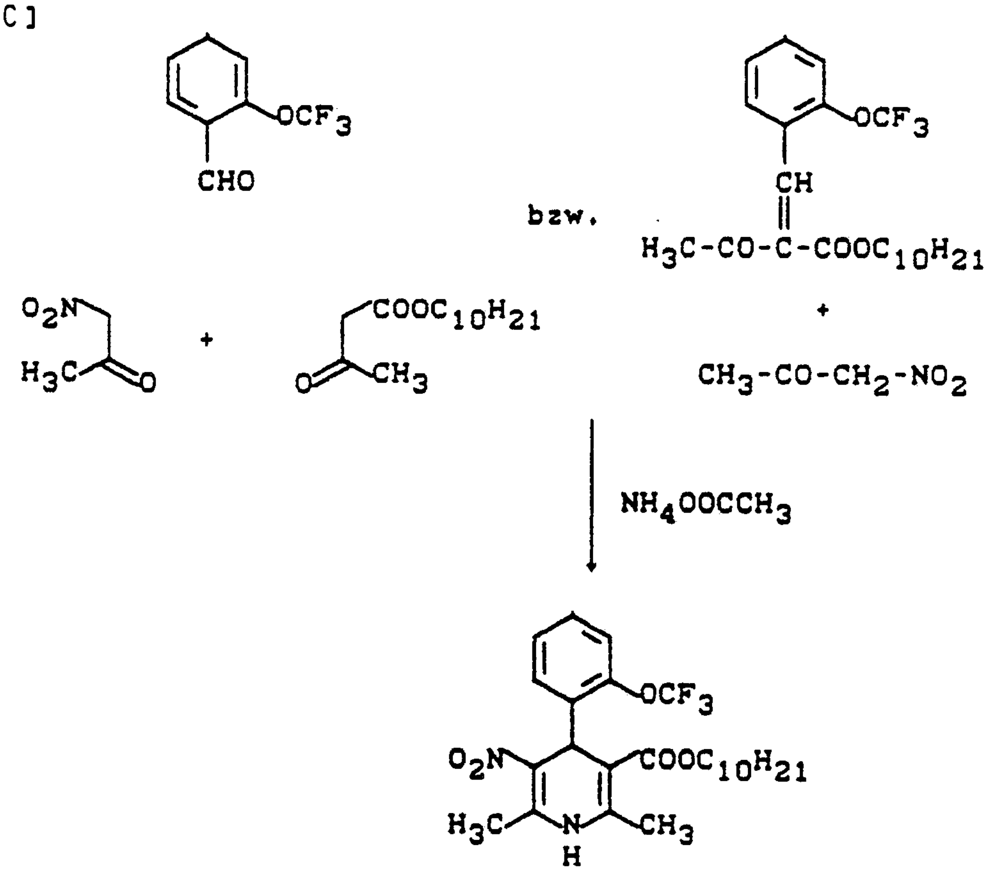


Die erfindungsgemäßen Verbindungen existieren in stereoisomeren Formen, die sich wie Bild und Spiegelbild (Enantiomere) verhalten. Die Erfindung betrifft sowohl die positiv inotrop wirksamen Enantiomeren als auch die Racemformen. Die Racemformen lassen sich in bekannter Weise in die stereoisomer einheitlichen Bestandteile trennen (vgl. E.L. Eliel, Stereochemistry of Carbon Compounds, McGraw Hill, 1962, oder DE-OS 3 447 169).

Als Verdünnungsmittel kommen für Verfahren A und C alle inerten organischen Lösemittel in Frage. Hierzu gehören bevorzugt Alkohole wie Methanol, Ethanol, n- bzw. iso-Propanol, Ether wie Diethylether, Tetrahydrofuran, Dioxan oder Glykolmono- oder diethylether, Eisessig, Pyridin, Dimethylformamid, Dimethylsulfoxid, Acetonitril oder Hexamethylphosphorsäuretriamid oder Toluol.

Für Verfahren B kommen die gleichen organischen Lösungsmittel, jedoch keine Alkohole, in Frage. Im Verfahren B ist es vorteilhaft, Decanole (Y = OH) mit ggf. reaktiven Dihydropyridinsäuren (VI) umzusetzen, oder die reaktiven Decylderivate mit Dihydropyridinsäuren oder deren Salzen umzusetzen.

Die Reaktionstemperaturen für Verfahren A bis C können in einem größeren Bereich variiert werden. Im allgemeinen arbeitet man in einem Bereich von 10° C bis 200° C, bevorzugt von 20° C bis 150° C.

Bei der Durchführung der erfindungsgemäßen Verfahren ist das Verhältnis der an der Reaktion beteiligten Stoffe beliebig. Im allgemeinen arbeitet man jedoch mit molaren Mengen der Reaktanden.

Die als Ausgangsstoff eingesetzten Fluormethoxybenzaldehyde (II) können nach in der Fluorchemie üblichen Methoden aus Salicylaldehyd und Chlorfluormethanen hergestellt werden.

Nitroaceton (III) ist bekannt [N. Levy, C.W. Scoufen, J. Chem. Soc. 1946, 1100; C.D. Huret, M.E. Nilson, J. Org. Chem. 20, 927 (1955)].

Die Ylidenverbindungen (IV) sind neu, können aber nach bekannten Methoden hergestellt werden (vgl. H. Dornow und W. Sassenberg, Liebigs Ann. Chem. 602, 14 (1957)).

Der Aminocrotonsäureester der Formel (V) ist bekannt [vgl. S.A. Glickman, A.C. Cope, J. Am. Chem. Soc. 67, 1017 (1945)].

Der erfindungsgemäße Acetessigester der Formel (VIII) ist bekannt [vgl. D. Borrmann, "Umsetzung von Diketen mit Alkoholen, Phenolen und Mercaptanen", in Houben-Weyl, Methoden der organischen Chemie, Vol. VII/4, 230 ff (1968)].

Die erfindungsgemäß verwendbaren Ylidenverbindungen der Formel (IX) sind bekannt oder können nach bekannten Methoden hergestellt werden [Organic Reactions XV, 204 ff, 1967].

Die DHP-Carbonsäuren (VI) sind neu und können nach bekannten Methoden hergestellt werden (vgl. EP 718 819).

Die vorstehenden Herstellungsverfahren sind lediglich zur Verdeutlichung angegeben. Die Herstellung der Ver bindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Die erfindungsgemäßen Verbindungen zeigen ein nicht vorhersehbares, wertvolles pharmakologisches Wirkspektrum. Sie beeinflussen die Kontraktionskraft des Herzens und den Tonus der glatten Muskulatur. Sie können deshalb in Arzneimitteln zur Beeinflussung des pathologisch veränderten Blutdrucks, als Koronartherapeutika und zur Behandlung der herzinsuffizienz eingesetzt werden. Darüber hinaus können sie zur Behandlung von Herzrhythmusstörungen, zur Senkung des Blutzuckers, zur Abschwellung von Schleimhäuten und zur Beeinflussung des Salz- und Flüssigkeitshaushaltes verwendet werden.

Die Herz- und Gefäßwirkungen wurden am isoliert perfundierten Herzen des Meerschweinchens gefunden.

Dazu werden die Herzen von 250 bis 350 g schweren Meerschweinchen verwendet. Die Tiere werden mit einem Schlag auf den Kopf getötet, der Thorax geöffnet, und in die freipräparierte Aorta eine Metallkanüle eingebunden. Das Herz wird mit den Lungen aus dem Thorax herausgetrennt und über eine Aortenkanüle an die Perfusionsapparatur bei laufender Perfusion angeschlossen. Die Lungen werden an den Lungenwurzeln abgetrennt. Als Perfusionsmedium dient eine Krebs-Henseleit-Lösung (1) (118,5 mmol/l Nacl, 4,75 mmol/l KCl, 1,19 mmol/l $KH_2PO_4$, 1,19 mmol/l $MgSO_4$, 25 mmol/l $NaHCO_3$, 0,013 mmol/l $Na_2$EDTA), deren $CaCl_2$-Gehalt 1,2 mmol/l beträgt. Als energielieferndes Substrat werden 10 mmol/l Glucose zugesetzt. Vor der Perfusion wird die Lösung partikelfrei filtriert. Die Lösung wird mit Carbogen (95% $O_2$, 5% $CO_2$) zur Aufrechterhaltung des pH-Wertes 7,4 begast. Die Herzen werden mit konstantem Fluß (10 ml/min) bei 32° C mittels einer Rollenquetschpumpe perfundiert.

Zur Messung der Herzfunktion wird ein flüssigkeitsgefüllter Latexballon, der über eine Flüssigkeitssäule mit einem Druckaufnehmer verbunden ist, durch den linken Vorhof in den linken Ventrikel eingeführt, und die isovolumetrischen Kontraktionen auf einem Schnellschreiber registriert (Opie, L., J. Physiol. 180 (1965), 529 -541). Der Perfusionsdruck wird mittels eines Druckaufnehmers, der vor dem Herzen mit dem Perfusionssystem in Verbindung steht, registriert. Unter diesen Bedingungen zeigt eine Senkung des Perfusionsdrucks eine Koronardilatation, eine Zu- bzw. Abnahme der linksventrikulären Kontraktionsamplitude eine Senkung bzw. einen Anstieg der Herzkontraktilität an. Die erfindungsgemäßen Verbindungen werden in geeigneten Verdünnungen in das Perfusionssystem kurz vor dem isolierten Herzen perfundiert.

Substanzeffekte auf die Kontraktionsamplitude isolierter Meerschweinchen-Herzvorhöfe bei einer Wirkstoffkonzentration von $10^{-3}$ g/l:

| Bsp.-Nr. | Änderung in % |
| --- | --- |
| 1 | +45 |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem medikament , der Art von dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falls der Applikation größerer Mengen kann es empfehlenswert sein, diese in mehreren Einzelgaben über den Tag zu verteilen.

## Herstellung

### Beispiel 1

#### Methode A

4-(2-Difluormethoxyphenyl)-1,4-dihydro-2,6-dimethyl-3-nitro-pyridin-5-carbonsäure-decylester

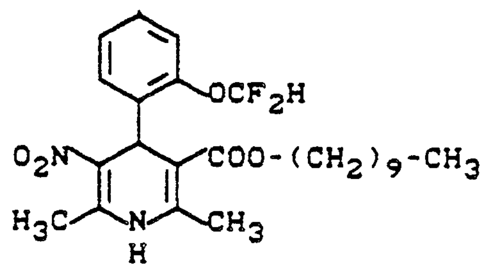

10,28 g (40 mmol) 2-Difluormethoxybenzyliden-nitroaceton werden in 50 ml Ethanol mit 9,5 g (40 mmol) ß-Aminocrotonsäuredecylester und 2,4 ml (40 mmol) Essigsäure 3 Stunden gekocht. Es wird eingeengt, der erhaltene ölige Eindampfrückstand wird in Essigester aufgenommen und mit Wasser, Natriumhydrogencarbonatlösung und wieder Wasser gewaschen, getrocknet und eingeengt. Der Eindampfrückstand wird mit Hexan/Ether 10:1 versetzt. Nach erfolgter Kristallisation wird abgesaugt und mit Hexan/Ether 10:1 gewaschen. Man erhält 17,5 g (91 % d. Th.) orangegelber Kristalle vom Schmelzpunkt 94-95 °C.

In analoger Weise wird hergestellt:

Beispiel 2

4-(2-Trifluormethoxyphenyl)-1,4-dihydro-2,6-dimethyl-3-nitro-pyridin-5-carbonsäure-decylester

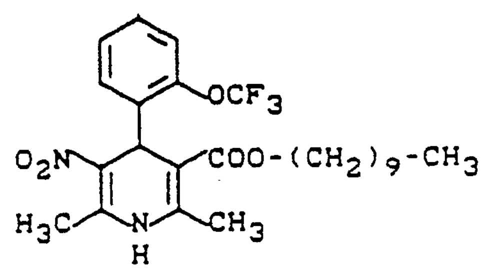

Schmp.: 108 °C

Beispiel 3 (Methode B)

4-(2-Difluormethoxyphenyl)-1,4-dihydro-2,6-dimethyl-3-nitro-pyridin-5-carbonsäure-decylester

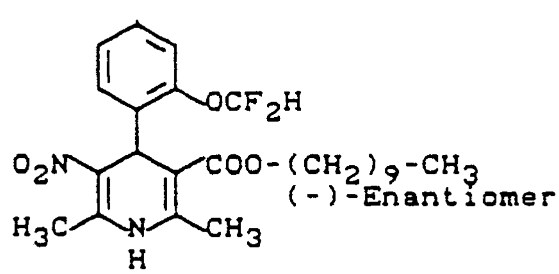

1,14 g (2,5 mmol) 4-(2-Difluormethoxyphenyl)-1,4-dihydro-2,6-dimethyl-3-nitro-pyridin-5-carbonsäure-1-(R)-(1-isopropoxycarbonyl-ethyl)-ester (Schmelzpunkt 167 °C) hergestellt aus 2-Difluormethoxy-benzylidennitroaceton und ß-Aminocorotonsäure-1-(R)-(1-isopropoxycarbonyl-ethyl)-ester, werden in 40 ml Dioxan und

50 ml 1,5N Natronlauge 48 Stunden gerührt. Nach Einengung wird zweimal mit Methylenchlorid ausgeschüttelt. Die wäßrige Phase wird mit 10 %iger Salzsäure angesäuert, zweimal mit Essigester ausgeschüttelt, mit Wasser gewaschen, getrocknet und eingeengt und das erhaltene Produkt über eine Kieselgelsäule gereinigt.

Das erhaltene (+)-Enantiomer der 4-(2-Difluormethoxy-phenyl)-1,4-dihydro-2,6-dimethyl-3-nitropyridin-5-carbon-säure ($[\alpha]^{20}_{589}$ = + 17,6 (c=0,512, Aceton)) wird in einer Menge von 1,5 g in 10 ml absolutem Dimethylformamid gelöst und nacheinander mit 10 g Decanol, 0,44 g 4-Dimethyl-aminopyridin und 2,55 g 1-Cyclohexyl-3-(2-morpholinoethyl)-carbondiimid-p-methoxy-toluolsulfonat 44 Stunden gerührt. Danach wird vom ausgefallenen Salz abgesaugt, das Filtrat eingeengt, der ölige Rückstand in Essigester aufgenommen, mit Wasser, 5%iger Salzsäure, Wasser, Natriumhydrogencarbonatlösung und wieder Wasser gewaschen, getrocknet und eingeengt. Der Eindampfrückstand wird durch Flash-Chromatografie mit Cyclohexan/Toluol-Gemischen gereinigt. Die Fraktionen werden eingeengt, durch Verrühren mit Ether/Heptan 1:10 kristallisiert und abgesaugt. Man erhält gelbe Kristalle vom Schmelzpunkt 87-88 °C.

$[\alpha]^{20}_{589}$ = -27,8 (c=0,71, Chloroform) (-)-Enantiomer.

In analoger Wiese wird hergestellt:

Beispiel 4

4-(2-Trifluormethoxyphenyl)-1,4-dihydro-2,6-dimethyl-3-nitro-pyridin-5-carbonsäure-decylester

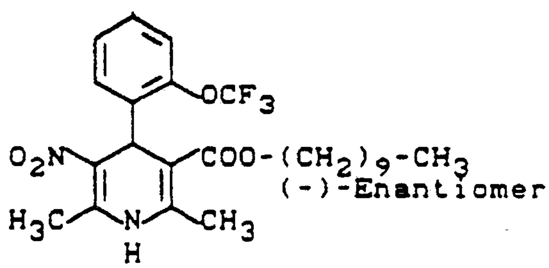

(-)-Enantiomer

Schmp.: 106 °C

$[\alpha]^{20}_{589}$ = -23,6 (Chloroform)

**Ansprüche**

1. Fluormethoxyphenyl-dihydropyridine der allgemeinen Formel (I)

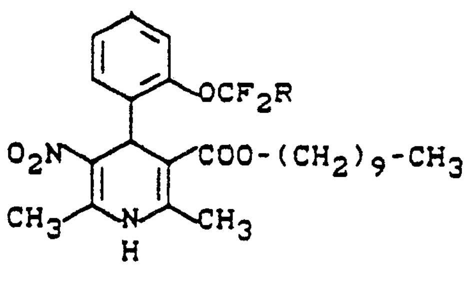

(I),

in welcher

R - für Wasserstoff oder Fluor steht.

2. Verbindungen der allgemeinen Formel (I) gemäß Anspruch 1 zur Bekämpfung bei Erkrankungen.

3. Verfahren zur Herstellung von Verbindungen der allgemeinen Formel (I)

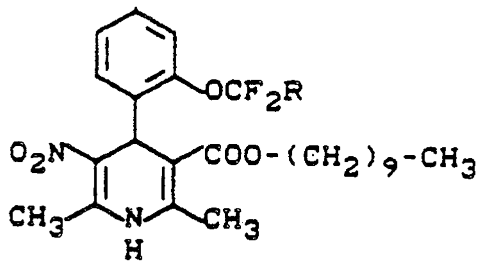

(I),

in welcher
R - für Wasserstoff oder Fluor steht,
dadurch gekennzeichnet, daß man
[A] den Aldehyd der Formel (II)

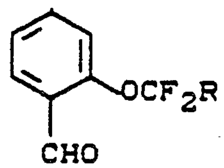

(II)

und Nitroaceton der Formel (III)

$H_3C-CO-CH_2-NO_2$ (III)

oder deren Knoevenagel-Kondensationsprodukt der Formel (IV),

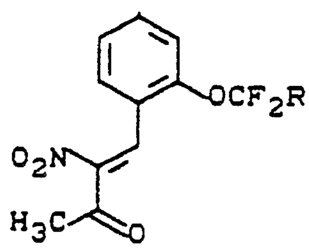

(IV)

in welcher R die oben angegebene Bedeutung hat,
und Aminocrotonsäureester der Formel (V)

$$CH_3-\underset{NH_2}{\underset{|}{C}}=CH=COO-(CH_2)_9-CH_3 \qquad (V)$$

gegebenenfalls in Anwesenheit inerter Lösemittel umsetzt,
oder indem man
[B] das Dihydropyridin der Formel (VI)

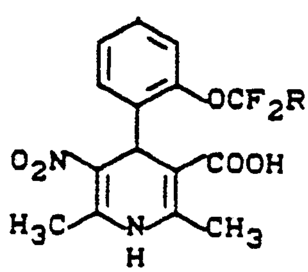

(VI)

in welcher
R die oben angegebene Bedeutung hat,

mit einem gegebenenfalls reaktiven Decanol der Formel (VII)

$H_3C-(CH_2)_9-Y$ (VII)

in welcher
Y für Hydroxyl oder eine aktivierende Gruppe wie Halogen, $-O-SO_2O-(CH_2)_9-CH_3$ oder ähnliche steht, nach bekannten Methoden, gegebenenfalls über ein reaktives Säurederivat, umsetzt,
oder indem man
[C] den Aldehyd der Formel (II) und den Acetessigester der Formel (VIII)

$H_3C-CO-CH_2COO-(CH_2)_9-CH_3$ (VIII)

bzw. deren Knoevenagel-Kondensationsprodukte der Formel (IX)

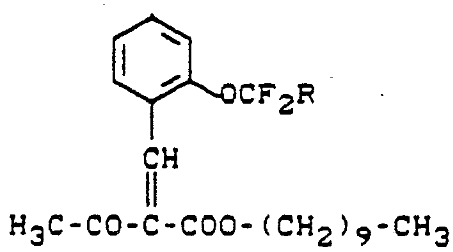

(IX)

in welchen
R die oben angegebene Bedeutung hat,
mit Nitroaceton und Ammoniumsalzen wie z.B. Ammoniumacetat, gegebenenfalls in Anwesenheit inerter Lösemittel, umsetzt und die erhaltenen Dihydropyridine gegebenfalls nach üblichen Methoden, z.B. durch Chromatographie über chirale stationäre Phasen auftrennt.

4. Verfahren gemäß Anspruch 3, dadurch gekennzeichnet, daß man die Umsetzungen und Auftrennungen in Gegenwart von inerten organischen Lösungsmitteln bei Temperaturen zwischen 10 und 200 °C durchführt.

5. Arzneimittel enthaltend mindestens eine Verbindung gemäß Anspruch 1.

6. Verfahren zur Herstellung von Arzneimitteln, dadurch gekennzeichnet, daß man eine Verbindung der allgemeinen Formel (I) gemäß Anspruch 1, gegebenenfalls unter Verwendung von üblichen Hilfs- und Trägerstoffen in eine geeignete Applikationsform überführt.

7. Verwendung von Verbindungen der allgemeinen Formel (I) gemäß Anspruch 1 bei der Herstellung von Arzneimitteln zur Beeinflussung des Kreislaufs, von Herzkrankheiten, des Zuckerhaushalts und des Salzund Flüssigkeitshaushalts.

8. Verwendung von Verbindungen der allgemeinen Formel (I) gemäß Anspruch 1 bei der Bekämpfung von Gefäßerkrankungen.